(19) Europäisches Patentamt / European Patent Office / Office européen des brevets

(11) **EP 2 103 298 A1**

(12) **EUROPEAN PATENT APPLICATION**

(43) Date of publication:
**23.09.2009 Bulletin 2009/39**

(21) Application number: **09002349.0**

(22) Date of filing: **19.02.2009**

(51) Int Cl.:
*A61K 8/02* (2006.01)     *A61K 8/33* (2006.01)
*A61K 8/34* (2006.01)     *A61Q 1/14* (2006.01)
*A61Q 19/10* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **21.03.2008 JP 2008072827**

(71) Applicant: **Daicel Chemical Industries, Ltd.
Kita-ku,
Osaka-shi
Osaka 530-0001 (JP)**

(72) Inventor: **Sakanishi, Yuichi
Ohtake-shi
Hiroshima 739-0695 (JP)**

(74) Representative: **Grünecker, Kinkeldey,
Stockmair & Schwanhäusser
Anwaltssozietät
Leopoldstrasse 4
80802 München (DE)**

(54) **Lamellar type liquid crystal composition for cosmetic**

(57)    Disclosed is a lamellar liquid crystal composition for cosmetic use, which contains one or more polyglycerol monoethers and has a lamellar liquid crystalline phase formed by the polyglycerol monoethers. The polyglycerol monoethers each have a hydrophile-lipophile balance (HLB) of 6 to 12 and are represented by following Formula (I):

$$RO\text{-}(GL)_n\text{-}H \qquad (I)$$

wherein R represents a linear or branched alkyl, alkenyl, or hydroxyalkyl group; "n" denotes an integer of from 2 to 10; and GL represents a glycerol unit. The cosmetic composition exhibits a superior cleansing power regardless of whether the skin is wet or not, and gives a satisfactory feel during use without leaving an oily sensation after rinsing with water.

**Description**

BACKGROUND OF THE INVENTION

1. Field of the Invention

The present invention relates to lamellar liquid crystal compositions for cosmetic use (hereinafter also referred to as "lamellar liquid crystal cosmetic compositions"), which contain one or more polyglycerol monoethers each having a specific structure and which satisfactorily work to remove makeup; and to cosmetics containing the cosmetic compositions.

2. Description of the Related Art

In the field of cosmetics, cleansing cosmetics are commercially available to remove stains or makeup (old makeup) from the skin. These cosmetics are classified as various formulations including those containing large amounts of oils, such as creams, milky lotions, oils, and oil-based gels; and those containing no or a trace amount of oils, such as lotions and water-borne gels.

However, when such a cleansing cosmetic containing large amounts of oils is used to remove makeup, the oil components remain on the skin after cleansing off the makeup from the skin, and the skin must be rinsed or cleansed again typically with a cleanser (face-washer). Additionally, a cleansing cosmetic of this type, if used for wet skin, exhibits an unsatisfactory cleansing power, gives an undesirable feel during use, and is unsuitable for use on the sweated skin or upon bathing.

In contrast, a cleansing cosmetic containing no or a trace amount of oils gives less oily sensation after cleansing, but the cleansing power thereof is insufficient.

To solve the problems, there have been proposed water-borne gel detergents containing polyoxyethylene fatty acid esters (see Japanese Unexamined Patent Application Publication (JP-A) No. Hei 4-5213; and JP-A No. Hei 8-143420). These water-borne gel detergents, however, do not exhibit a sufficient cleansing power, and their component polyoxyethylene surfactants cause problems in the safety. Additionally, they are insufficiently stable during storage due to the fatty acid moiety in the molecule.

To solve the problems in safety, there have been developed various techniques. Typically, there have been proposed cleansing cosmetics containing polyglycerol fatty acid esters or polyglycerol monoalkyl ethers (see JP-A No. 2007-23025; and JP-A No. 2006-347900). However, the cleansing cosmetics containing polyglycerol fatty acid esters, when formulated into water-borne cleansing cosmetics, may undergo hydrolysis and may not be so stable over time. On the other hand, polyglycerol monoalkyl ethers, if formulated into cleansing cosmetics, do not form a lamellar liquid crystalline phase and the resulting cleansing cosmetics are not sufficiently resistant to water.

SUMMARY OF THE INVENTION

Accordingly, an object of the present invention is to provide a cosmetic composition which is useful typically in a cleansing cosmetic that is able to be mixed with makeup stains (old makeup) easily on the skin regardless of whether the skin is wet (moistened) or not. Another object of the present invention is to provide a cosmetic using the cosmetic composition.

Yet another object of the present invention is to provide a cosmetic composition which is useful typically in a cosmetic that does not leave an oily sensation after rinsing off with water, is satisfactorily stable, is satisfactorily rinsed off with water, and exhibits these superior properties stably over time. Still another object of the present invention is to provide a cosmetic using the cosmetic composition.

After intensive investigations to achieve the objects, the present inventors have found that use of a specific polyglycerol monoether gives a cosmetic composition forming a lamellar liquid crystalline phase. They have further found that a cleansing cosmetic using the cosmetic composition is capable of mixing well with makeup stains (old makeup) regardless of whether the skin is wet or not, enables the oil makeup to be lifted up rapidly, is satisfactorily stable, and is satisfactorily rinsed off with water.

Specifically, according to an embodiment of the present invention, there is provided a lamellar liquid crystal cosmetic composition, which includes one or more polyglycerol monoethers and has a lamellar liquid crystalline phase formed by the one or more polyglycerol monoethers, in which the one or more polyglycerol monoethers each have a hydrophile-lipophile balance (HLB) of 6 to 12 and are each represented by following Formula (I):

$$RO\text{-}(GL)\text{ n-H} \qquad (I)$$

wherein R represents a linear or branched alkyl, alkenyl, or hydroxyalkyl group; "n" denotes an integer of from 2 to 10; and GL represents a glycerol unit.

The lamellar liquid crystal cosmetic composition according to the present invention may further contain one or more organic compounds having two or more hydroxyl groups per molecule.

The lamellar liquid crystal composition according to the present invention is preferably used for a cleansing cosmetic.

According to another embodiment of the present invention, there is further provided a cosmetic containing the lamellar liquid crystal cosmetic composition.

Lamellar liquid crystal cosmetic compositions according to the present invention are capable of mixing with makeup stains easily or old makeup and exhibit a superior cleansing power regardless of whether the skin is wet or not.

Additionally, they give a satisfactory clean feel during use without leaving an oily sensation after rinsing with water.

These and other objects, features, and advantages of the present invention will be understood more fully from the following detailed description of the preferred embodiments. All numbers are herein assumed to be modified by the term "about."

DESCRIPTION OF THE PREFERRED EMBODIMENTS

Polyglycerol monoethers for use herein are polyglycerol monoethers which have a HLB of 6 to 12 and are represented by Formula (I), in which R is a linear or branched alkyl, alkenyl, or hydroxyalkyl group; and "n" denotes an integer of from 2 to 10.

Of these groups, R is preferably a linear or branched alkyl, alkenyl, or hydroxyalkyl group having eight to eighteen carbon atoms and is more preferably a linear or branched alkyl, alkenyl, or hydroxyalkyl group having twelve to eighteen carbon atoms.

Exemplary preferred alkyl groups include octyl group, nonyl group, decyl group, undecyl group, dodecyl group, tridecyl group, tetradecyl group, pentadecyl group, hexadecyl group, heptadecyl group, octadecyl group, isohexadecyl group, isotetradecyl group, and isooctadecyl group.

Exemplary preferred alkenyl groups include octenyl group, nonenyl group, decenyl group, undecenyl group, dodecenyl group, tridecenyl group, tetradecenyl group, pentadecenyl group, hexadecenyl group, heptadecenyl group, and octadecenyl group.

Exemplary preferred hydroxyalkyl groups include hydroxyoctyl group, hydroxynonyl group, hydroxydecyl group, hydroxyundecyl group, hydroxydodecyl group, hydroxytridecyl group, hydroxytetradecyl group, hydroxypentadecyl group, hydroxyhexadecyl group, hydroxyheptadecyl group, and hydroxyoctadecyl group.

The repetition number "n" in Formula (I) is an integer of from 2 to 10, and is preferably an integer of from 2 to 5.

GL in Formula (I) represents a glycerol unit. The glycerol unit has a structure of following Formula (II) or Formula (III):

$$-CH_2-CHOH-CH_2O- \qquad (II)$$

$$-CH(CH_2OH)CH_2O- \qquad (III)$$

Polyglycerol monoethers for use herein are those having a hydrophile-lipophile balance (HLB) of 6 to 12. The hydrophile-lipophile balance (HLB) is a value indicating the balance between hydrophilicity and lipophilicity of surfactants. As used herein a hydrophile-lipophile balance (HLB) refers to a value calculated according to the following equation from an "organic value" and an "inorganic value" determined by the organic conception diagram.

$$\mathtt{HLB\ =\ (Inorganic\ value)/(Organic\ value)\ x\ 10}$$

The HLB of a polyglycerol monoether can be controlled by suitably selecting R and the repetition number "n" in combination.

The way to prepare such polyglycerol monoethers is not particularly limited, and exemplary preparation processes include a process of carrying out a reaction by adding glycidol to an aliphatic alcohol in the presence of a basic catalyst so that the molar ratio of the alcohol to glycidol in the reaction mixture being a specific value; a process of reacting an $\alpha$-olefin epoxide with a polyglycerol; and a process of opening the ring of an alkyl glycidyl ether using a polyglycerol in the presence of an acid catalyst or an alkali catalyst.

Exemplary preferred polyglycerol monoethers for use herein include diglycerol monododecyl ether, triglycerol monododecyl ether, triglycerol monotetradecyl ether, tetraglycerol monotetradecyl ether, triglycerol monohexadecyl ether, tetraglycerol monotetradecyl ether, tetraglycerol monooctadecyl ether, tetraglycerol monoisostearyl ether, and tetraglycerol monooleyl ether. Among them, more preferred examples are diglycerol monododecyl ether, tetraglycerol monotetradecyl ether, tetraglycerol monoisostearyl ether, and tetraglycerol monooleyl ether.

The amount of polyglycerol monoethers is not particularly limited but is preferably 0.01 to 80 percent by weight, more preferably 0.1 to 50 percent by weight, further preferably 5 to 45 percent by weight, and particularly preferably 15 to 40 percent by weight, based on the total amount of the lamellar liquid crystal cosmetic composition. Each of different polyglycerol monoethers can be used alone or in combination.

Lamellar liquid crystal cosmetic compositions according to embodiments of the present invention have satisfactory affinity for the skin and help the skin to retain moisture satisfactorily effectively, because they each have a lamellar liquid crystalline phase that is very close to the structure of lipids present between keratinocytes.

The lamellar liquid crystal cosmetic compositions according to embodiments of the present invention preferably further contain one or more organic compounds having two or more hydroxyl groups per molecule.

Of organic compounds having two or more hydroxyl groups per molecule, polyhydric alcohols are preferred. Cosmetic compositions, if further containing such polyhydric alcohols, act to solubilize dramatically increased amounts of oils and water, because the polyhydric alcohols effectively act to retain liquid crystals in the lamellar liquid crystalline phase.

Of polyhydric alcohols, preferred are those each having three to fifteen carbon atoms and two to twelve hydroxyl groups per molecule.

Exemplary polyhydric alcohols include glycerol, diglycerol, maltitol, 1,3-butylene glycol, isoprene glycol, propylene glycol, dipropylene glycol, polyethylene glycols, pentaerythritol, neopentyl glycol, sorbitol, sorbitan, trehalose, and polypropylene glycols. Among them, glycerol, maltitol, 1,3-butylene glycol, propylene glycol, and sorbitol are desirably used. Each of different polyhydric alcohols can be used alone or in combination.

The content of polyhydric alcohols is preferably 7 to 70 percent by weight, more preferably 10 to 50 percent by weight, and particularly preferably 12 to 40 percent by weight, based on the total amount of the lamellar liquid crystal cosmetic composition. Lamellar liquid crystal cosmetic compositions, if containing polyhydric alcohols in a content within the above range, are further highly stable and can be satisfactorily rinsed off with water.

The ratio (weight ratio) of polyglycerol monoethers to polyhydric alcohols in the lamellar liquid crystal cosmetic compositions according to embodiments of the present invention is preferably from 90:10 to 10:90 and more preferably 80:20 to 40:60.

In a preferred embodiment of the present invention, a lamellar liquid crystal cosmetic composition further contains one or more liquid paraffins. Such liquid paraffins are formulated in order to act typically as protecting agents or solvents, because they have emollient actions and moisturizing actions and are highly conformable with the skin to cover the skin satisfactorily. The amount of liquid paraffins can be suitably set and is, for example, preferably 3 to 30 percent by weight, more preferably 7 to 20 percent by weight, and particularly preferably 7 to 15 percent by weight, based on the total amount of the lamellar liquid crystal cosmetic composition.

In a lamellar liquid crystal cosmetic composition according to a preferred embodiment, water is used as a liquid solvent. The amount of water can be suitably set and is, for example, 10 to 95 percent by weight, preferably 25 to 70 percent by weight, and more preferably 30 to 60 percent by weight, based on the total amount of the lamellar liquid crystal cosmetic composition.

The lamellar liquid crystal cosmetic compositions may be in any form. For example, they may be lotions, solutions, milky lotions, creams, or gels. They are preferably creams or gels, because such creams or gels are easy to use.

Where necessary, the lamellar liquid crystal cosmetic compositions may further contain other ingredients to be used in regular cosmetics, in addition to the above ingredients. Exemplary other ingredients include nonionic surfactants other than the specific polyglycerol monoethers, as well as anionic surfactants, amphoteric surfactants, alcohols, lower alcohols, powders, antioxidants, antioxidation assistants, ultraviolet-absorbers, humectants, anti-inflammatory agents, preservatives, pH-adjusters, extracts derived from animals, vegetables, fishes and shellfishes, microorganism and flavors.

Other nonionic surfactants than the specific polyglycerol monoethers are not particularly limited, but are preferably surfactants having no ionizable group as a hydrophilic group. Examples of such surfactants include glycerol fatty acid esters, polyglycerol fatty acid esters, polyalkylene glycol fatty acid esters, sorbitan fatty acid esters, sugar fatty acid esters, pentaerythritol fatty acid esters, polyoxyalkylene hydrogenated castor oil fatty acid esters, fatty acid alkanolamides, polyoxyalkylene glycols, esters between a polyoxyalkylene glycol and a monohydric or polyhydric alcohol, polyoxyalkylene sugar ethers, condensates between a fatty acid amide and a polyoxyalkylene glycol, condensates between an aliphatic amine and a polyoxyalkylene glycol, and alkyl or alkenyl polyglucosides.

Exemplary anionic surfactants include, but are not limited to, salts of polyoxyethylene alkyl ether sulfuric acid, salts of alkyl sulfuric acid esters, salts of alkylbenzenesulfonic acids, salts of α-olefin-sulfonic acids, glutamic acid and other amino acid surfactants, salts of N-acylmethyltaurines, and salts of alkyl phosphates.

Exemplary amphoteric surfactants include, but are not limited to, various amphoteric surfactants such as carboxybetaine compounds, imidazolinium compounds, sulfobetaine compounds, and alanine compounds.

Exemplary lower alcohols include, but are not limited to, ethanol and propyl alcohol.

Powders (powdery components) include, but are not limited to, inorganic powders and organic powders. Exemplary inorganic powders include talc, kaolin, mica, sericite, muscovite, phlogopite, synthetic mica, lepidolite, biotite, vermiculite, magnesium carbonate, zirconium silicate, aluminum silicate, barium silicate, calcium silicate, zinc silicate, magnesium silicate, strontium silicate, metal tungstates, magnesium, silica, zeolite, barium sulfate, calcined calcium sulfate (calcined gypsum), calcium phosphate, fluoroapatite, hydroxyapatite, ceramic powders, activated carbon, medical carbon (medical charcoal), metal soaps (e.g., zinc myristate, calcium palmitate, and aluminum stearate), and boron nitride. Exemplary organic powders include polyamide resin powders (nylon powders), polyethylene powders, poly (methyl methacrylate) powders, polystyrene powders, powders of styrene-acrylic acid copolymers, benzoguanamine resin powders, and cellulose powders.

Exemplary antioxidants include, but are not limited to, vitamin E, dibutylhydroxytoluene, butylhydroxyanisole, and gallic acid esters. Exemplary antioxidation assistants include, but are not limited to, ascorbic acid, phytic acid, kephalin, and maleic acid.

Exemplary ultraviolet-absorbers include, but are not limited to, benzophenone derivatives such as 2-hydroxy-4-methoxybenzophenone, 2-hydroxy-4-methoxybenzophenone-5-sulfonic acid, salts of 2-hydroxy-4-methoxybenzophenone-5-sulfonic acid, and benzophenone derivative such as dihydroxydimethoxybenzophenone; p-aminobenzoic acid derivatives such as p-aminobenzoic acid and ethyl p-aminobenzoate; methoxycinnamic acid derivatives such as ethyl p-methoxycinnamate, isopropyl p-methoxycinnamate, and octyl p-methoxycinnamate; salicylic acid derivatives such as octyl salicylate and phenyl salicylate; urocanic acid and derivatives thereof; 4-tert-butyl-4'-methoxydibenzoylmethane; 2-(hydroxy-5'-methylphenyl)benzotriazole; and methyl o-aminobenzoate (methyl anthranilate).

Exemplary humectants include, but are not limited to, sodium lactate, pyrrolidonecarboxylic acid, and pyrrolidonecarboxylic acid derivatives.

Exemplary anti-inflammatory agents include, but are not limited to, glycyrrhizic acid and derivatives thereof, glycyrrhetic acid and derivatives thereof, allantoin, hydrocortisone acetate, and azulene.

Exemplary preservatives include, but are not limited to, methylparaben (methyl p-hydroxybenzoate), propylparaben (propyl p-hydroxybenzoate), and phenoxyethanol.

Exemplary pH adjusters include, but are not limited to, citric acid, hydrochloric acid, sulfuric acid, phosphoric acid, sodium hydroxide, and ammonia.

Exemplary extracts derived from animals, vegetables, fishes and shellfishes, or microorganisms include, but are not limited to, extracted components such as tea extract, aloe extract, ginkgo extract, swertia herb extract, mugwort extract, garlic extract, Scutellaria root extract, rosemary extract, sponge gourd extract, placental extract, extract from lactic acid bacteria culture, and seaweed extract.

Flavors for use herein are not particularly limited, as long as being those generally used in cosmetics.

Cosmetic compositions according to embodiments of the present invention are used in a variety of cosmetics such as cleansing cosmetics and massage cosmetics and are particularly advantageously used in cleansing cosmetics for removing stains and old makeup from the skin.

Cosmetics according to embodiments of the present invention can be prepared typically by dissolving the ingredients with heating, mixing them, and cooling the mixture. However, the ways to prepare the cosmetics are not limited, as long as being known or common preparation processes.

Examples

The present invention will be illustrated in further detail with reference to several examples below. It should be noted, however, that these are illustrated only by way of example and are never construed to limit the scope of the present invention.

A series of cleansing cosmetic compositions was prepared by mixing ingredients according to the formulations of Examples 1 to 3 in Table 1 and the formulations of Comparative Examples 1 to 3 in Table 2. The properties of the prepared cleansing cosmetic compositions were evaluated according to the criteria below. The results are shown in Table 3.

(Evaluations)

[Cleansing Power (unwet condition)]

A lipstick was applied to the forearm, about 0.5 g of a sample cosmetic composition was taken in hand, and the portion bearing the applied lipstick was massaged with the cosmetic composition on the hand 30 times. How the lipstick came off was visually observed, and the cleansing power of the sample cosmetic composition was evaluated according to the following criteria:

[Criteria for Cleansing Power (unwet condition)]

A: Lipstick fully comes off
B: Lipstick substantially comes off
C: Lipstick remains slightly
D: Lipstick substantially remains (substantially no lipstick comes off)

[Liquid Crystal Form (unwet condition)]

A sample cosmetic composition was observed under a polarization microscope, and whether lamellar liquid crystals were formed or not was determined.

[Cleansing Power (wet condition)]

A lipstick was applied to the forearm, the forearm was wetted with water, about 0.5 g of a sample cosmetic composition was taken in hand, and the portion bearing the applied lipstick was massaged by the cosmetic composition on the hand 30 times. How the lipstick came off was visually observed, and the cleansing power of the sample cosmetic composition was evaluated according to the following criteria.

[Criteria for Cleansing Power (wet condition)]

A: Lipstick fully comes off
B: Lipstick substantially comes off
C: Lipstick remains slightly
D: Lipstick substantially remains (substantially no lipstick comes off)

[Liquid Crystal Form (wet condition)]

A sample cosmetic composition (0.5 g) was thoroughly mixed with 0.3 g of water, the mixture was observed under a polarization microscope, and whether lamellar liquid crystals were formed or not was determined.

TABLE 1

| Formulation (percent by weight) | Example 1 | Example 2 | Example 3 |
| --- | --- | --- | --- |
| Water | 42 | 42 | 42 |
| Glycerol | 18 | 18 | 18 |
| Diglycerol monododecyl ether (HLB: 9.4) | 30 | | |
| Tetraglycerol monoisostearyl ether (HLB: 9.7) | | 30 | |
| Tetraglycerol monooleyl ether (HLB: 9.7) | | | 30 |
| Liquid paraffin | 10 | 10 | 10 |

TABLE 2

| Formulation (percent by weight) | Comparative Example 1 | Comparative Example 2 | Comparative Example 3 |
| --- | --- | --- | --- |
| Water | 42 | 42 | 42 |
| Glycerol | 18 | 18 | 18 |

(continued)

| Formulation (percent by weight) | Comparative Example 1 | Comparative Example 2 | Comparative Example 3 |
|---|---|---|---|
| Tetraglycerol monododecyl ether (HLB: 12.1) | 30 | | |
| Hexaglycerol monododecyl ether (HLB: 13.7) | | 30 | |
| Decaglycerol monododecyl ether (HLB: 15.5) | | | 30 |
| Liquid paraffin | 10 | 10 | 10 |

TABLE 3

| Evaluation | Ex. 1 | Ex. 2 | Ex. 3 | Com. Ex. 1 | Com. Ex. 2 | Com. Ex. 3 |
|---|---|---|---|---|---|---|
| Cleansing power (unwet) | A | A | A | B | B | B |
| Lamellar liquid crystals (unwet) | Formed | Formed | Formed | None | None | None |
| Cleansing power (wet) | A | A | A | C | D | D |
| Lamella liquid crystals (wet) | Formed | Formed | Formed | None | None | None |

As demonstrated by the above results, cosmetic compositions according to embodiments of the present invention each have a lamellar liquid crystalline phase, can mix with makeup stains (old makeup) easily, thereby exhibit a superior cleansing power regardless of whether the skin is wet or not, and give a satisfactory feel during use without leaving an oily sensation after rinsing with water.

While the above description is of the preferred embodiments of the present invention, it should be appreciated that the invention may be modified, altered, or varied without deviating from the scope and fair meaning of the following claims.

## Claims

1.   A lamellar liquid crystal composition for cosmetic use, comprising one or more polyglycerol monoethers and having a lamellar liquid crystalline phase formed by the one or more polyglycerol monoethers, wherein the one or more polyglycerol monoethers each have a hydrophile-lipophile balance (HLB) of 6 to 12 and are each represented by following Formula (I):

$$RO\text{-}(GL)n\text{-}H \qquad (I)$$

wherein R represents a linear or branched alkyl, alkenyl, or hydroxyalkyl group; "n" denotes an integer of from 2 to 10; and GL represents a glycerol unit.

2.   The lamellar liquid crystal composition according to claim 1, further comprising one or more organic compounds having two or more hydroxyl groups per molecule.

3.   The lamellar liquid crystal composition according to one of claims 1 and 2, for a cleansing cosmetic.

4.   A cosmetic comprising the lamellar liquid crystal composition of any one of claims 1 to 3.

**EUROPEAN SEARCH REPORT**

Application Number

EP 09 00 2349

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 62 205187 A (POLA CHEM IND INC) 9 September 1987 (1987-09-09) * the whole document * | 1-4 | INV. A61K8/02 A61K8/33 A61K8/34 A61Q1/14 A61Q19/10 |
| X | JP 2007 146029 A (LION CORP) 14 June 2007 (2007-06-14) * paragraph [0024] - paragraph [0029]; claims; examples * | 1-4 | |
| X | JP 2006 282895 A (TAIYO KAGAKU KK) 19 October 2006 (2006-10-19) * the whole document * | 1,3,4 | |
| X | JP 2006 282894 A (TAIYO KAGAKU KK) 19 October 2006 (2006-10-19) * the whole document * | 1,3,4 | |
| X | JP 62 204839 A (POLA CHEM IND INC) 9 September 1987 (1987-09-09) * the whole document * | 1,3,4 | |
| X | EP 1 428 515 A (OREAL [FR]) 16 June 2004 (2004-06-16) * claims; examples * | 1-4 | TECHNICAL FIELDS SEARCHED (IPC) A61K A61Q |
| X | JP 04 005213 A (KAO CORP) 9 January 1992 (1992-01-09) * the whole document * | 1-4 | |
| D,X | JP 2006 347900 A (TAIYO KAGAKU KK) 28 December 2006 (2006-12-28) * the whole document * | 1-4 | |
| X | JP 05 239492 A (KAO CORP) 17 September 1993 (1993-09-17) * the whole document * | 1-4 | |

-/--

The present search report has been drawn up for all claims

| Place of search | Date of completion of the search | Examiner |
|---|---|---|
| Munich | 16 July 2009 | Donovan-Beermann, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**Europäisches Patentamt**
**European Patent Office**
**Office européen des brevets**

# EUROPEAN SEARCH REPORT

Application Number

EP 09 00 2349

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| P,X | JP 2008 094812 A (NARIS COSMETICS CO LTD) 24 April 2008 (2008-04-24) * the whole document * ----- | 1-4 | |
| E | JP 2009 073787 A (MANDOM CORP) 9 April 2009 (2009-04-09) * the whole document * ----- | 1-4 | |
| A | CA 2 610 440 A1 (TAIYO KAGAKU KK [JP]) 21 December 2006 (2006-12-21) * the whole document * | 1 | |
| D,A | & JP 2007 023025 A (TAIYO KAGAKU KK) 1 February 2007 (2007-02-01) ----- | 1-4 | |
| A | JP 08 143420 A (PROCTER & GAMBLE) 4 June 1996 (1996-06-04) * the whole document * ----- | 1 | |

TECHNICAL FIELDS
SEARCHED    (IPC)

The present search report has been drawn up for all claims

| Place of search | Date of completion of the search | Examiner |
|---|---|---|
| Munich | 16 July 2009 | Donovan-Beermann, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 09 00 2349

This annex lists the patent family members relating to the patent documents cited in the above-mentioned European search report.
The members are as contained in the European Patent Office EDP file on
The European Patent Office is in no way liable for these particulars which are merely given for the purpose of information.

16-07-2009

| Patent document cited in search report | | Publication date | Patent family member(s) | | Publication date |
|---|---|---|---|---|---|
| JP 62205187 | A | 09-09-1987 | NONE | | |
| JP 2007146029 | A | 14-06-2007 | NONE | | |
| JP 2006282895 | A | 19-10-2006 | NONE | | |
| JP 2006282894 | A | 19-10-2006 | NONE | | |
| JP 62204839 | A | 09-09-1987 | NONE | | |
| EP 1428515 | A | 16-06-2004 | AT 399527 T<br>FR 2848437 A1 | | 15-07-2008<br>18-06-2004 |
| JP 4005213 | A | 09-01-1992 | NONE | | |
| JP 2006347900 | A | 28-12-2006 | NONE | | |
| JP 5239492 | A | 17-09-1993 | JP 2716904 B2 | | 18-02-1998 |
| JP 2008094812 | A | 24-04-2008 | NONE | | |
| JP 2009073787 | A | 09-04-2009 | NONE | | |
| CA 2610440 | A1 | 21-12-2006 | WO 2006134886 A1 | | 21-12-2006 |
| JP 2007023025 | A | 01-02-2007 | NONE | | |
| JP 8143420 | A | 04-06-1996 | JP 2849339 B2 | | 20-01-1999 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI45213 A
- JP HEI8143420 A
- JP 2007023025 A
- JP 2006347900 A